# EUROPEAN PATENT APPLICATION

(11) **EP 4 241 798 A1**
(43) Date of publication of application: **13.09.2023**
(21) Application number: 22382231.3
(22) Date of filing: 11.03.2022
(51) Int. Cl.: A61L 27/22, A61L 27/26, A61L 27/52

(54) **HYDROGEL SUITABLE TO PRODUCE ARTIFICIAL TISSUES**

(71) Applicant: Universidad de Granada, 18071 Granada (ES)
(72) Inventor: GILA VÍLCHEZ, Cristina, 18071 Granada (ES); MAÑAS TORRES, María del Carmen, 18071 Granada (ES); CARRIEL ARAYA, Víctor Sebastián, 18071 Granada (ES); ÁLVAREZ DE CIENFUEGOS RODRÍGUEZ, Luis, 18071 Granada (ES); ALAMINOS MINGORANCE, Miguel, 18071 Granada (ES); LÓPEZ LÓPEZ, Modesto Torcuato, 18071 Granada (ES); GARCÍA GARCÍA, Óscar Darío, 18071 Granada (ES)
(74) Representative: Hoffmann Eitle

(57) **Abstract**

The present invention refers to a method for the preparation of a hydrogel suitable to produce artificial tissues. The present invention also covers a biomaterial or artificial tissue obtained by implementing said method. Finally, the present invention is directed to the use of the biomaterial or artificial tissue thus obtained as a medicament, preferably for the regeneration of tissues or organs, more preferably for restoring or partially or fully replacing the functional activity of a diseased or damaged tissue or organ.

## Description

### FIELD OF THE INVENTION

The present invention refers to the medical field. Particularly, the present invention refers to a method for the preparation of a hydrogel suitable to produce artificial tissues. The present invention also covers a biomaterial or artificial tissue obtained by implementing said method. Finally, the present invention is directed to the use of the biomaterial or artificial tissue thus obtained as a medicament, preferably for the regeneration of tissues or organs, more preferably for restoring or partially or fully replacing the functional activity of a diseased or damaged tissue or organ.

### STATE OF THE ART

The technique behind the present invention is named tissue engineering. Tissue engineering is an interdisciplinary field that applies the principles of biology and engineering to the development of strategies for the replacement, repair, maintenance and/or enhancement of biological tissues. Three different approaches within tissue engineering can be distinguished: (i) exclusive use of cells; (ii) exclusive use of polymeric matrices; and (iii) combination of cells and polymeric matrices. The present invention falls under approach (iii). In this approach, regeneration of biological tissues is achieved by using polymeric matrices in combination with cells and growth factors that enhance cell adhesion and proliferation, so that the resulting biomaterial can replace the native material, fulfilling its physiological functions in a satisfactory manner. In the resulting biomaterial, as in a native tissue, all vital functions emanate from the cellular machinery and the interaction between adjacent cells.

The extracellular matrix (polymeric matrix in an artificial biomaterial) also plays an essential role as it provides the necessary support for the proliferation of cells and the maintenance of their functions. An ideal polymeric matrix should meet the following characteristics: (i) it should be three-dimensional and highly porous, with interconnected pores to facilitate cell growth and nutrient transport; (ii) it should have adequate surface chemical properties to allow cell adhesion, proliferation and differentiation; (iii) it should have biomechanical properties appropriate to the native tissue it is intended to replace. This last characteristic is particularly important in the case of load-bearing tissues such as those of the musculoskeletal system, such as cartilage and bone. As an example, note that only 3-5% of the volume of cartilage is made up of cells (chondrocytes), which have mechanical stiffness moduli several orders of magnitude lower than those of the extracellular matrix, and therefore do not contribute appreciably to the mechanical properties of cartilage as a whole

Materials used in tissue engineering for polymeric matrix generation include collagen, fibrin, alginate, agarose, chitosan and hyaluronic acid. They have the advantage of being biocompatible and biodegradable, and of providing suitable physiological conditions for cell adhesion and proliferation. However, they all have disadvantages such as lack of a well-organised and controlled internal structure, biomechanical weakness and rapid degradation *in vivo.* These are all critically important limitations that must be overcome in order to maximise the development of tissue engineering applications of these materials.

So, there is an unmet medical need of finding reliable strategies for producing materials to be used in tissue engineering which, apart from being biocompatible, biodegradable and provide suitable physiological conditions for cell adhesion and proliferation, they also have a well-organised and controlled internal structure and biomechanical strength which avoid a rapid degradation *in vivo.*

The present invention is thus focused on solving this technical problem and an innovative method for the preparation of a hydrogel suitable to produce artificial tissues is herein provided.

### DESCRIPTION OF THE INVENTION

### Brief description of the invention

The present invention refers to a method for the preparation of a hydrogel suitable to produce artificial tissues, to a biomaterial or artificial tissue obtained by implementing said method and, finally, it also refers to the use of the biomaterial or artificial tissue thus obtained as a medicament, preferably for the regeneration of tissues or organs, more preferably for restoring or partially or fully replacing the functional activity of a diseased or damaged tissue or organ.

The biomaterial or artificial tissue of the invention is a completely new biocompatible material that improves the biomechanical properties described in the prior art for other similar products. The biomaterial or artificial tissue of the invention combines both biocompatibility and improved mechanical properties. The biomaterial or artificial tissue of the invention is not only novel in its composition, but also possesses improved mechanical and biological properties by combining fibrin or fibrinogen with the appropriate peptides. This difference originates from the interaction between the fibrin or fibrinogen and the peptides, which results in the appearance of a more suitable internal structure while providing better biocompatibility. In existing works, hydrogels present a series of limitations that differentiate them, to a greater or lesser degree, from the native extracellular matrices they are intended to mimic, such as the lack of a well-organised and controlled internal structure, or their weakness from a biomechanical point of view. However, the present invention is based on the use of fibrin, combined with peptides that provide a new structure and biocompatibility. In this way, a change in the internal structure of the material is produced, which in turn causes a change in its biomechanical properties.

In fact, many of the artificial tissues prepared by tissue engineering, and in particular those prepared using fibrin and agarose, have mechanical properties suitable for use as replacements for most native human tissues. However, getting the right porosity and internal structure for a proper cell growth at the same time is a big challenge. For example, while the addition of agarose helps to improve the mechanical properties of the material compared to a fibrin hydrogel, the porosity of the material decreases as the agarose concentration increases, which is an impediment to its subsequent use as a cell growth promoting material. In this sense, the strategy followed in the present invention, comprising the introduction of short-chain peptides, improves both aspects, increasing the mechanical strength of the material and obtaining a suitable internal structure for its use as an artificial tissue. Moreover, although agarose is biocompatible, it is also inert from the point of view of cell adhesion and proliferation. However, the composition of the present invention allows the inclusion of specific peptides that improve cell adhesion and proliferation of the tissue to be regenerated.

So, the first embodiment of the present invention refers to a method for the preparation of a hydrogel suitable to produce artificial tissues (hereinafter *method of the invention),* which comprises combining the following components:
a. Fibrin, fibrinogen or any source of fibrin or fibrinogen such as the human plasma;
b. An antifibrinolytic agent;
c. A fibrinogen polymerisation inducing agent;
d. A peptide with the ability to self-aggregate to form β-sheets;
e. A peptide biological ligand; and
f. Water.

In a preferred embodiment of the present invention, the peptide with the ability to self-aggregate to form β-sheets is a short-chain amino acid peptide with a molecular mass up to 2 kDa.

In a preferred embodiment of the present invention, the peptide with the ability to self-aggregate to form β-sheets is selected from the list comprising: Fmoc-FF (Fmoc-diphenylalanine), Fmoc-AA (Fmoc-dialanine), Fmoc-GG (Fmoc-diglycine), Fmoc-FA (Fmoc-phenylalanine-alanine), Fmoc-FG (Fmoc-phenylalanine-glycine), Fmoc-LG (Fmoc-leucine-glycine), Fmoc-YT (Fmoc-tyrosine-threonine), Fmoc-FY (Fmoc-phenylalanine-tyrosine), Fmoc-YS (Fmoc-tyrosine-serine), Fmoc-CF (Fmoc-cysteine-phenylalanine) or Fmoc-YN (Fmoc-tyrosine-asparagine).

In a preferred embodiment of the present invention, the peptide biological ligand is selected from the list comprising: Fmoc-RGD (Fmoc-arginine-glycine-aspartate), Fmoc-RGDA (Fmoc-arginine-glycine-aspartate-alanine), Fmoc-RGDS (Fmoc-arginine-glycine-aspartate-serine), Fmoc-RGDF (Fmoc-arginine-glycine-aspartate-phenylalanine) or Fmoc-FRGD (Fmoc-phenylalanine-arginine-glycine-aspartate).

In a preferred embodiment of the present invention, the peptide with the ability to self-aggregate to form β-sheets is Fmoc-FF and the peptide biological ligand is Fmoc-RGD.

It is highly important to consider that, in the present invention, a specific ratio between the fibrin or fibrinogen and the peptide components has been defined, which is specially suitable for obtained the product of the invention. This ratio is preferably at least 3:1, most preferably 6:1. In a preferred embodiment, this ratio is between 3:1 (e.g. at least 3 volume units of fibrin or fibrinogen by every volume unit of peptide components) and 99:1 (v/v) (e.g. at least 99 volume units of fibrin or fibrinogen by every volume unit of peptide components), with a most preferable ratio of 6:1 (v/v).

In a preferred embodiment of the present invention, the fibrinogen polymerisation inducing agent is a clotting factor, thrombin, a source of calcium preferably a calcium salt most preferably calcium chloride, or any combination thereof.

In a preferred embodiment of the present invention, the antifibrinolytic agent is tranexamic acid.

In a preferred embodiment, the present invention further comprises the addition of a protein, being a peptide with a chain of amino acids having a minimum molecular weight of 4 kDa, preferably fibronectin.

In a preferred embodiment of the present invention, the fibrin or fibrinogen is incorporated into the mixture by adding blood plasma.

In a preferred embodiment of the present invention, the blood plasma is of autologous origin.

In a preferred embodiment of the present invention, the method comprises the sequential execution of the following steps:
a) Adding an antifibrinolytic agent to a composition comprising fibrin or fibrinogen;
b) Adding at least one fibrinogen polymerisation inducing agent to the product resulting from step (a);
c) Adding a composition comprising a peptide with the ability to self-aggregate into sheets to the product resulting from step (b);
d) (d) adding a composition comprising a peptide biological ligand to the product resulting from step (c); and
e) Adding water.

In a preferred embodiment of the present invention, the method comprises adding a sample of biological material, preferably a sample comprising a plurality of isolated cells, or culturing the cells inside the resulting product.

In a preferred embodiment of the present invention, the cells are cells of epithelial, connective, muscular or nervous lineage.

In a preferred embodiment of the present invention, the cells are fibroblasts and/or keratinocytes.

In a preferred embodiment of the present invention, the cells cultured inside the resulting product comprise mesenchymal stem cells, preferably from umbilical cord, bone marrow, adipose tissue or dental pulp.

In a preferred embodiment of the present invention, the cells cultured inside the resulting product comprises epithelial cells or cells with epithelial differentiation capacity.

In a preferred embodiment of the present invention, the epithelial cells are selected from the list comprising: keratinocytes of the skin or oral mucosa, urothelial cells, urethral epithelial cells, corneal epithelial or endothelial cells, vascular endothelial cells, digestive tract epithelial cells, respiratory epithelial cells, urogenital epithelial cells and other types of epithelial cells.

In a preferred embodiment of the present invention, the cells incorporated into the biological material or cultured inside the resulting material are of autologous origin.

In a preferred embodiment of the present invention, the cells incorporated into the biological material or cultured inside the resulting material are fibroblasts originating from the stroma of a tissue or organ selected from the list comprising: oral mucosa, abdominal wall, skin, bladder, urethra or cornea.

The second embodiment of the present invention refers to a biomaterial or artificial tissue (hereinafter *biomaterial or artificial tissue of the invention)* obtainable by the method of the invention.

In a preferred embodiment of the present invention, the biomaterial or artificial tissue of the invention comprises fibrin, a peptide with the ability to self-aggregate to form β-sheets and peptide biological ligand.

In a preferred embodiment of the present invention, the biomaterial or artificial tissue of the invention comprises a peptide with the ability to self-aggregate to form β-sheets which is a short-chain amino acid peptides with a molecular mass up to 2 kDa.

In a preferred embodiment of the present invention, the biomaterial or artificial tissue of the invention comprises a peptide with the ability to self-aggregate to form β-sheets selected from the list comprising: Fmoc-FF, Fmoc-AA, Fmoc-GG, Fmoc-FA, Fmoc-FG, Fmoc-LG, Fmoc-YT, Fmoc-FY, Fmoc-YS, Fmoc-CF or Fmoc-YN

In a preferred embodiment of the present invention, the biomaterial or artificial tissue of the invention comprises a peptide biological ligand selected from the list comprising: Fmoc-RGD Fmoc-RGDA, Fmoc-RGDS, Fmoc-RGDF or Fmoc-FRGD.

In a preferred embodiment of the present invention, the biomaterial or artificial tissue of the invention comprises a peptide with the ability to self-aggregate to form β-sheets which is Fmoc-FF and a peptide biological ligand which is Fmoc-RGD.

In a preferred embodiment of the present invention, the biomaterial or artificial tissue of the invention comprises a ratio between the fibrin or fibrinogen and the peptide components up to 3:1.

In a preferred embodiment of the present invention, the biomaterial or artificial tissue of the invention comprises a ratio between the fibrin or fibrinogen and the peptide components of 6:1.

The third embodiment of the present invention refers to a composition (hereinafter *composition of the invention)* comprising the biomaterial or artificial tissue of the invention.

The fourth embodiment of the present invention refers to an implant (hereinafter *implant of the invention)* comprising the biomaterial or artificial tissue of the invention or the composition of the invention.

The fifth embodiment of the present invention refers to a pharmaceutical composition (hereinafter *pharmaceutical composition of the invention)* comprising the biomaterial or artificial tissue of the invention and optionally pharmaceutically acceptable vehicles or carriers.

In a preferred embodiment of the present invention, the pharmaceutical composition comprises another active substance.

The sixth embodiment of the present invention refers to the biomaterial or artificial tissue of the invention, the composition of the invention, the implant of the invention or the pharmaceutical composition of the invention, for use as a medicament, preferably for use in tissue or organ regeneration, more preferably for use in increasing, restoring or partially or fully replacing the functional activity of a diseased or damaged tissue or organ. Alternatively, the present invention refers to a method for regenerating tissues or organs, more preferably for increasing, restoring or partially or fully replacing the functional activity of a diseased or damaged tissue or organ, which comprises the administration or medial use of the composition of the invention, the implant of the invention or the pharmaceutical composition of the invention.

For the purpose of the present invention the following terms are defined:
- The term "comprising" means including, but not limited to, whatever follows the word "comprising". Thus, use of the term "comprising" indicates that the listed elements are required or mandatory, but that other elements are optional and may or may not be present.
- The term "consisting of' means including, and limited to, whatever follows the phrase "consisting of'. Thus, the phrase "consisting of' indicates that the listed elements are required or mandatory, and that no other elements may be present.

### Description of the figures

**Figure 1****.** SEM images of **(a)** fibrin and **(b)** fibrin-peptide (6:1) hydrogels **(c)** FTIR and **(d)** CD spectroscopy analyses.
**Figure 2****.** **(a)** Mass and **(b)** height of the hydrogels after the application of a normal force. Macroscopic appearance of freshly prepared hydrogels **of (c)** fibrin, **(d)** fibrin-FmocFF (6:1) and **(e)** fibrin-FmocFF (3:1).
**Figure 3****.** **(a)** Loss tangent as a function of time during gelation starting from mixtures of pregel reagents. **(b)** Mean values of storage modulus (G') within the LVR and **(c)** shear modulus (G) for nanostructured hydrogels. **(d)** Young modulus (E) for hydrogels before and after nanostructuration. **(e)** Amplitude sweeps and **(f)** frequency sweeps for pristine hydrogels under IN normal forces.
**Figure 4****.** **(a)** Microscopic images of cells from Live/Dead assays. Statistical analysis of **(b)** number of living cells from Live/Dead assays and **(c)** WST-1 assays.

### Detailed description of the invention

The present invention is illustrated by means of the examples set below without the intention of limiting its scope of protection.

### Example 1. Material and Methods

### Example 1.1. Peptides

N-Fluorenylmethoxycarbonyl diphenylalanine (Fmoc-FF) and N-Fluorenylmethoxycarbonyl Arginylglycylaspartic acid (Fmoc-RGD) peptides were purchased from Bachem Co. (Switzerland) and were used without further purification. Sodium hydroxide (NaOH), calcium chloride (CaCl₂), phosphate buffered saline (PBS) and Dulbecco's Modified Eagle's Medium (DMEM) were provided for Sigma Aldrich, USA. The anti-fibrinolytic agent (Amchafibrin) was purchased from Fides-Ecofarma, Valencia, Spain).

### Example 1.2. Preparation of peptide solutions

The Fmoc-FF solution was prepared following the next protocol. The desired amount of Fmoc-FF was weighed and MiliQ water was added in order to obtain a final solution of 20mM concentration. The resulting suspension was sonicated in a cold ultrasonic bath (AL04-03-230) until a homogeneous suspension was obtained after approximately 1 hour. Then, a solution of 0.5M NaOH was added in drops of 10 in 10 µL using a micropipette and sonicating for 2 minutes just after the addition of each drop. A clear solution was obtained at a pH of approximately 10.5.

On the other hand, Fmoc-RGD solution was prepared by adding the proper amount of Fmoc-RGD powder to MiliQ water and stirring until a clear 20mM solution was obtained.

For the preparation of hydrogels containing the biological ligand, Fmoc-FF / Fmoc-RGD solutions were mixed at a ratio 7/3.

### Example 1.3. Preparation of uncompressed and nanostructured fibrin-peptide hydrogels

For the preparation of uncompressed fibrin-peptide hydrogels, we proceeded as it follows. Fibrin hydrogels were prepared by mixing 3.8mL of human plasma, 875µL of DMEM, 75µL of Amchafibrin and 250µL of a 2% CaCl₂ solution in PBS (to promote fibrin gelation) for a final volume of 5mL. In the case of fibrin-peptide hydrogels, this precursor of fibrin hydrogels was mixed with the peptide solution and an additional amount of DMEM (to promote Fmoc-FF gelation) equal to the volume of peptide, at different proportions (see Table 1). Finally, the mixture was left at rest overnight at 37°C of temperature to ensure the complete gelation.

To prepare the nanostructured hydrogels, they were subjected to plastic compression techniques. Thus, the hydrogels were placed between a pair of nylon filter membranes of 0.22 µm pore size (Merck-Millipore, Darmstadt, Germany) and a pair of absorbent pieces of paper. Then, hydrogels were compressed for 3 minutes under a uniform mechanical pressure of 500 g homogeneously distributed.

**Table 1**

| Sample (5mL) | Volume of fibrin precursor | Volume of peptide solution^{∗}# | Volume of additional DMEM |
|---|---|---|---|
| Fibrin | 5mL | 0 | 0 |
| Fibrin-Peptide (6:1) | 4.25mL | 357 µL | 357µL |
| Fibrin-Peptide (3:1) | 3.75mL | 625 µL | 625 µL |

| | | | |
|---|---|---|---|
| *Summary of the main preparation proportions of the hydrogels of the present work.* ^{∗}Peptide concentration was 20 mM in all cases. ^{#}Ratio of Fmoc-FF to Fmoc-RGD was 7/3. | | | |

### Example 1.4. Physicochemical characterization of the hydrogels

### Scanning Electron Microscopy of the hydrogels

We analyzed the microstructure of hydrogels by Scanning Electron Microscopy (SEM) by means of a FEI Quanta 400 SEM. The hydrogels were previously prepared according to a well-established protocol in order to subject them to CO₂ critical point drying (Anderson, 1951) and coat them with Au-Pd (Ion Sputtering method).

### Fourier transform infra-red (FTIR) spectroscopy

We recorded FTIR spectra by using a Perkin-Elmer Two FTIR ATR spectrometer. The hydrogels were compressed onto the diamond crystal and the spectra were scanned over the wavenumber range from 4000 to 450 cm⁻¹.

### Circular Dichroism (CD)

The hydrogels were gelified into a 0.1 mm quartz cell (Hellma 0.1 mm quartz Suprasil) and the CD spectra were measured using a Jasco J-815 spectropolarimeter equipped with an air cooled 150 W Xenon lamp. A constant temperature of 20 °C was maintained during the measurements using a PFD-425 Peltier controller. The spectra was recorded from 200 to 350 nm with a step of 1 nm and 0.5 s of integration time per step. For each experimental condition, we took the average value of 100 measurements.

### Example 1.5. Mechanical evaluation of the hydrogels

### Gel kinetics

We investigated the gel kinetics of hydrogels by means of rheological measurements, using a Haake MARS III controlled-stress rheometer (Thermo Fisher Scientific, Waltham, MA, USA) provided with a double cone-plate sensor of 60 mm of diameter and 2° apex angle (sensor DC60/2° Ti L). For this aim we followed the protocol for the preparation of the hydrogels described above and poured the mixture in the measuring system of the rheometer. Then we subjected the gelling sample to oscillatory shear strain of fixed frequency (1 Hz) and amplitude (γ₀=0.001), monitoring the resulting viscoelastic moduli as a function of time and at a constant temperature of 37°C. The amplitude used in our work is low enough to ensure that the building of the gel microstructure was unperturbed.

### Characterization of the mechanical properties of hydrogels

We characterized the mechanical properties of the hydrogels under oscillatory shear strains by using a Haake MARS III controlled-stress rheometer (Thermo Fisher Scientific, Waltham, MA, USA) provided with a double plate sensor of 35 mm of diameter and rough surfaces to avoid wall slip (sensor P35 Ti L S serrated, Thermo Fisher Scientific, Waltham, MA, USA). Characterization was carried out at a constant temperature of 37.0 ± 0.1 °C. Firstly, we subjected the hydrogels to amplitude sweeps, for which the frequency of oscillation was kept at 1 Hz and the amplitude of the oscillatory strain, γ₀, was increased stepwise from 0.0001 to 2. From these measurements we obtained the values of the storage (G') and loss (G") moduli as a function of γ₀. Afterwards, we performed frequency sweep tests, for which the amplitude of the shear strain was fixed at γ₀=0.001, and the frequency of oscillation was increased stepwise from 0.1 to 16 Hz. From these measurements we obtained the values of G' and G" as a function of frequency (i.e., the mechanical spectra) of the hydrogels. In order to analyze the shear modulus (G) of these hydrogels, we applied shear stress sweeps from 0.1 to 300 Pa.

Finally, we characterized the mechanical properties of the hydrogels under tensile strains by using a Discovery Hybrid Rheometer (TA Instruments). For this purpose, we fixed the hydrogels with the rheometer clamps and subjected them to increasing axial strains at a constant velocity until the sample was broken and at room temperature. Then, we analyzed the Young Modulus obtained from these experiments.

A fresh sample was used for each test (amplitude and frequency sweeps) and experimental condition and performed measurements for at least three different aliquots of the same experimental condition. In this manuscript we provide the corresponding mean values and standard error of the measurements.

### Example 1.6. Cell viability assessment

First, a dispersion of human skin-derived fibroblasts (passage 13) from healthy donors in DMEM was seeded on the bottom of culture wells at a density of 15 ·103 cells per culture well and kept in culture for 24h. Then, the hydrogels were put in contact with the cultured cells and kept in contact for 48h. Finally, hydrogels were removed, and cells were subjected to Live/Dead Cell Viability Assay (Viability/Cytotoxicity kit; Molecular Probes, Eugene, Oregon, USA) and water-soluble tetrazolium salt-1 (WST-1) assay. In all cases, hydrogels without cells seeded on the bottom of the culture well were used as control. In addition, cells seeded on chamber slides without hydrogels contact were used as positive technical control, while cells seeded on chamber slides and incubated with 2% Triton X-100 were used as technical negative control. For the statistical analysis, three samples of each condition were studied.

To perform the Live/Dead Cell Viability Assay (LD), culture wells with cells seeded on bottom were rinsed in PBS three times (5min each) and then incubated with the working solution for 30min. They were then rinsed in PBS and analyzed under a Nikon Eclipse Ti fluorescence and light inverted microscope (Nikon) equipped with a Nikon DXM 1200c Digital Camera (Nikon).

In a similar way, after cells incubation and once the hydrogels and the medium were removed, the culture wells were rinsed in PBS and then were incubated with the WST-1 (Cell Proliferation Reagent WST-1, Roche Diagnostics, Mannheim, Germany) working solution for 4h at 37 °C.

### Example 2. Results

### Example 2.1. Physicochemical characterization of the hydrogels

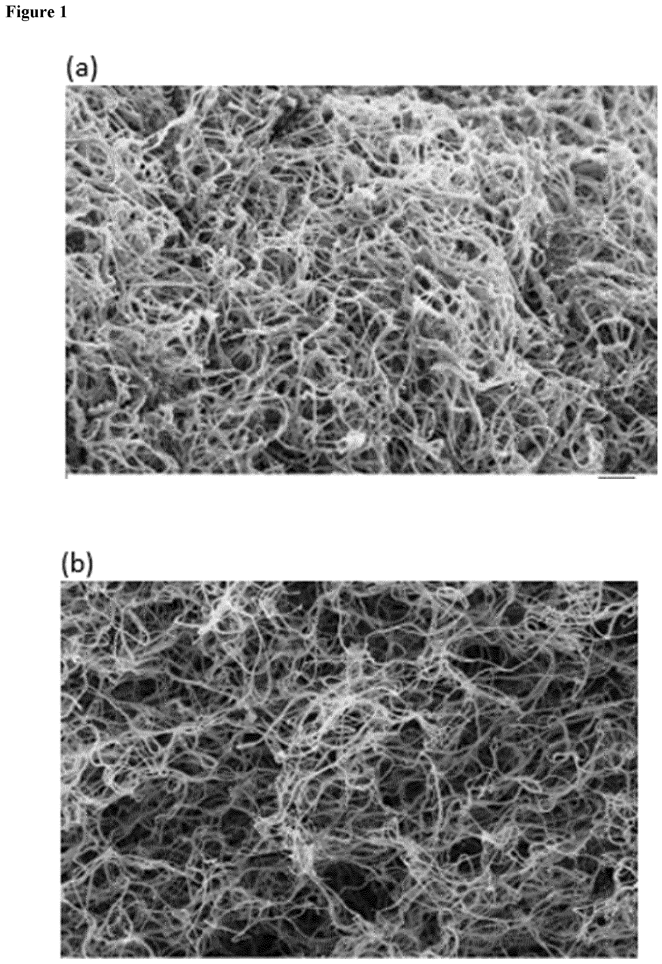

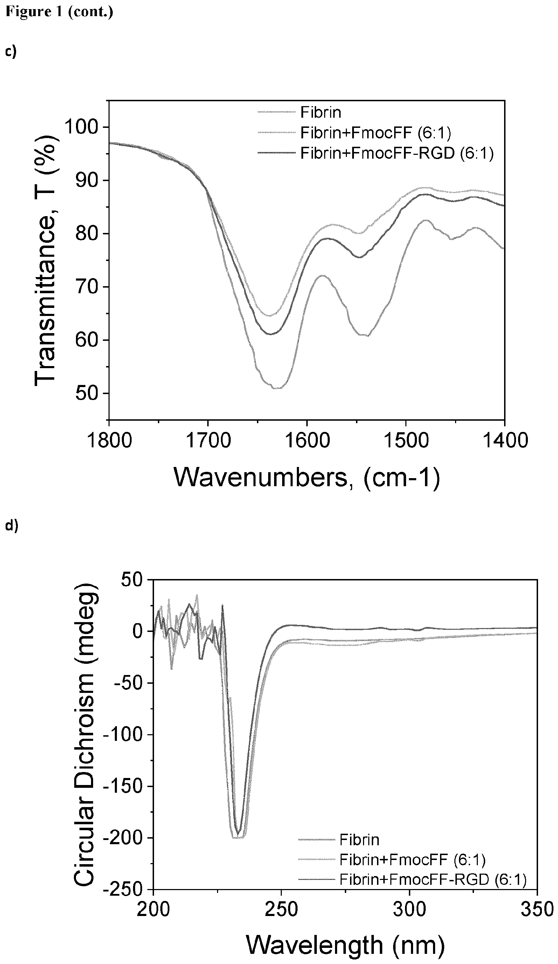

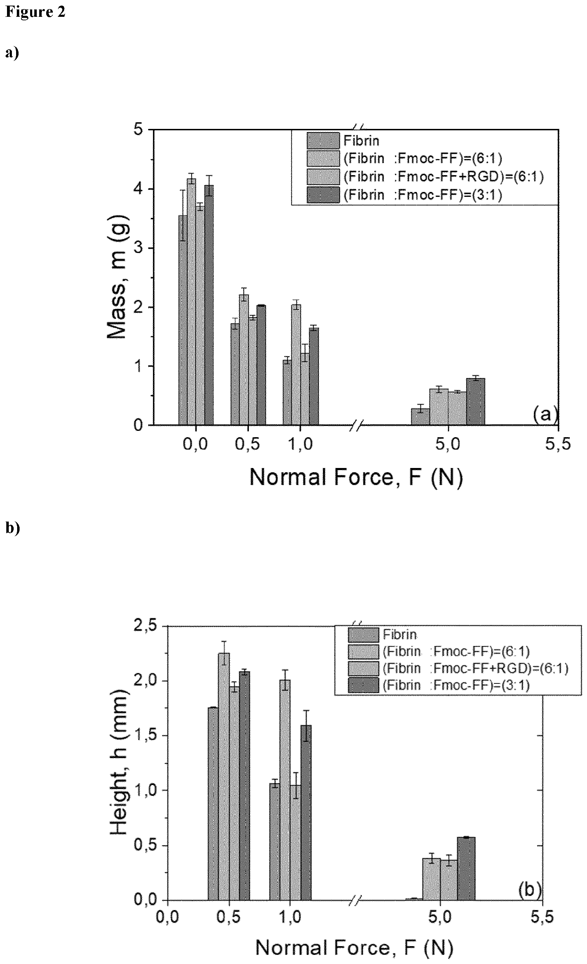

First, SEM images of fibrin and fibrin-peptide hydrogels (**Fig**. **1 a-b****)** showed a porous 3D network formed by fibrils. Both hydrogels with and without peptides in their composition showed a very similar internal structure and fibrils morphology, which indicates that the peptides are well integrated into the fibrin fibrils. However, an increase of the porosity is observed when peptides are included in the hydrogel's composition. Note that a higher density of fibers appears in the **Fig. 1a** compared with that of the **Fig. 2b****.** Thus, the inclusion of peptides enhances the hydrogels porosity without changing the inner structure of the fibrin fibers.

Further analysis of the secondary structure of these hydrogels as well as the capacity of fibrin and peptides to interact with each other was studied by FTIR **(****Fig. 1c****)** and CD **(Fig. Id)** spectra. FTIR spectroscopy of fibrin-peptide hydrogels showed practically superimposed spectra for all the mixtures and almost identical to the fibrin spectra alone **(****Fig. 1c****)**, which is dominated by intensive amide I at 1633 cm⁻¹ and amide II at 1541 cm⁻¹ bands. These intense amide bands correspond to the formation of structures due to the protein containing fibrin, while another lower contribution band corresponding to lipids appears at 1454 cm⁻¹. In comparison, those hydrogels containing peptides present a decrease on the intensity of these three bands, with the 1454 cm⁻¹ band almost absent. In the same way, the CD spectra of the fibrin-peptide hydrogels looked all almost identical to the spectra of the fibrin hydrogel alone. In all cases, the spectra presented a main negative band in the far ultraviolet region around 235 nm. This result goes in the line with the observed by FTIR considering that these supramolecular peptides, similar to proteins, have CD bands in the far ultraviolet region (210-240 nm) derived mainly from the amide groups and related to the secondary structure of the fibers.

Therefore, the analysis of the data obtained by SEM, FTIR and CD are all in agreement if we consider that the peptides are interacting with the fibrin, giving rise to fibers of similar morphological characteristics.

### Example 2.2 Mechanical evaluation of the hydrogels

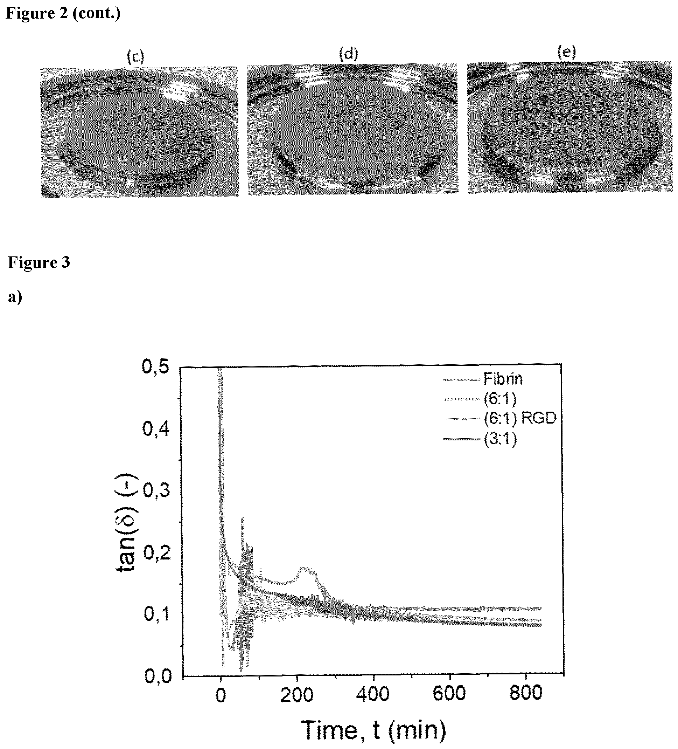

Firstly, we studied the changes in mass and height of the hydrogels after the application of different normal forces. It can be observed that initial mass of hydrogels including peptides is a little bit higher than those of only fibrin. This tendency is maintained after the application of the different normal forces, having greater differences as the normal force increases, with the unique exception of fibrin-peptide hydrogels including Fmoc-RGD and for the lower normal forces **(****Fig. 2a****).** Similar situation occurs for the height of the hydrogels, while in this case the differences between fibrin hydrogels only and fibrin-peptide hydrogels is even greater **(****Fig. 2b****).** These results go in the line with the macroscopic appearance of the hydrogels **(****Fig. 2c****-e),** in which we observe that fibrin-peptide hydrogels present a better consistency than those with only fibrin. This can be also related with SEM observations, as the higher porosity given by peptides can make them to retain more liquid and to show higher mass, better consistency and resistance to external forces.

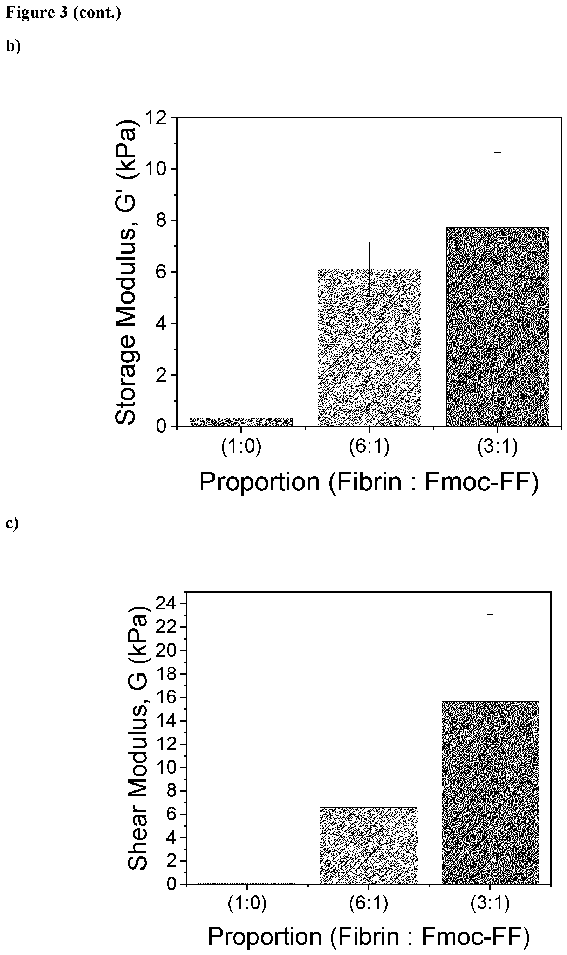

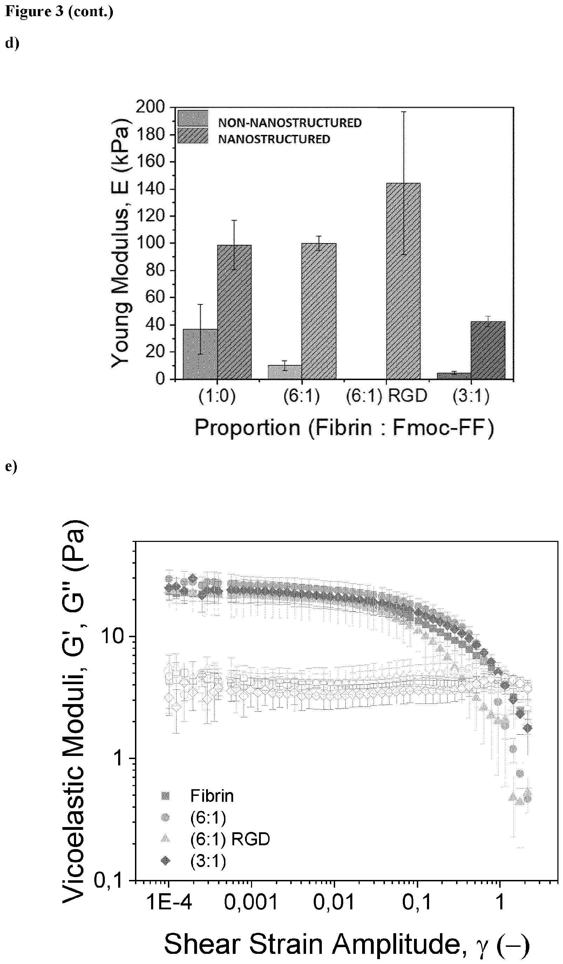

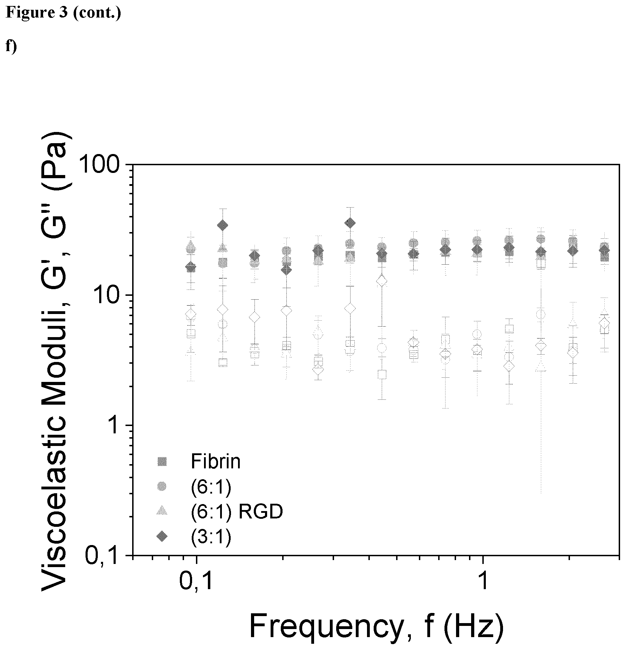

Then, we monitored the evolution of the storage (G') and loss (G") moduli as a function of time in order to study the gel kinetics of the hydrogels. In all cases, we observed a sharp increase of the viscoelastic moduli related to a fast gel formation as well as a gel-like character at the very beginning of the experiments, followed of a slower increase after a few minutes related with a deep gel formation **(****Fig. 3a****).** Thus, at low times of gelation, we obtain weak gels with values of tan(*δ*)=G"/G' between 1 and 0.1. In addition, the curves of the loss tangent evidence that fibrin hydrogels complete gelation is faster compared with those which contain peptides in their composition. However, fibrin hydrogels present final values of tan(*δ*) ≈ 0.1 whereas fibrin-peptide hydrogels present values of tan(*δ*) < 0.1, corresponding thus to a typical behavior of strong gels compared to those of only fibrin **(****Fig. 3a****).**

We also characterized the mechanical properties of hydrogels under oscillatory shear after complete gelation. We can identify two different regions on amplitude sweeps **(****Fig. 2e****).** The first region, which is commonly known as linear viscoelastic region (LVR), is characterized by values of G' and G" almost independent of the shear strain amplitude. At this region, the hydrogels under study are characterized by values of G' > G", as expected for solid-like materials. Then, at larger values of the shear strain amplitude a smooth rise in G" up to a maximum value, known as yield point, together with a smooth decrease in G', marked the onset of the nonlinear viscoelastic region (NLVR). Within the NLVR takes place a strong decrease of the viscoelastic moduli which is related to the destruction of the gel-like network, followed by a transition to a liquid-like behavior (G' < G"). We also carried out frequency sweeps for shear strain amplitude within the VLR **(Fig. 2f)** which showed almost frequency-independent trends for G', typical of densely crosslinked materials, while G" showed a small tendency to increase.

Whereas pristine hydrogels show small differences on their mean values of the storage modulus (G') within the linear viscoelastic region (LVR) **(****Fig. 2e****),** after plastic compression nanostructured hydrogels which contain peptides in their formulation reach G' values of an order of magnitude greater than those of only fibrin **(****Fig. 2b****).** In addition, the shear modulus (G) of these hydrogels goes from 0.13 kPa for fibrin nanostructured hydrogels to 16 kPa for those containing peptides, i.e., the inclusion of peptides enhances the shear modulus of the nanostructured hydrogels in two orders of magnitude **(****Fig. 2c****).**

Finally, we characterized the mechanical properties of hydrogels under static tensile stresses in order to obtain the Young modulus (E) of each hydrogel both, with and without nanostructuration. In this case, the Young modulus decreases when peptides were included for pristine hydrogels while more similar values were obtained after nanostructuration **(****Fig 2d****).** Note that high values of E≈100 kPa were obtained for fibrin-peptide hydrogels (6:1) and even higher to those with Fmoc-RGD.

All these results are in agreement with the macroscopic observations and the physicochemical characterization discussed above.

### Example 2.3. Ex vivo evaluation of the hydrogels

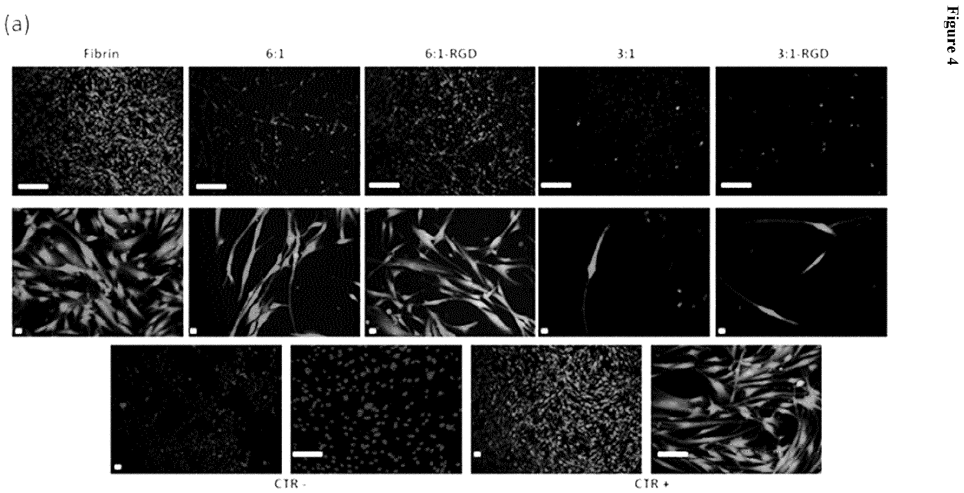

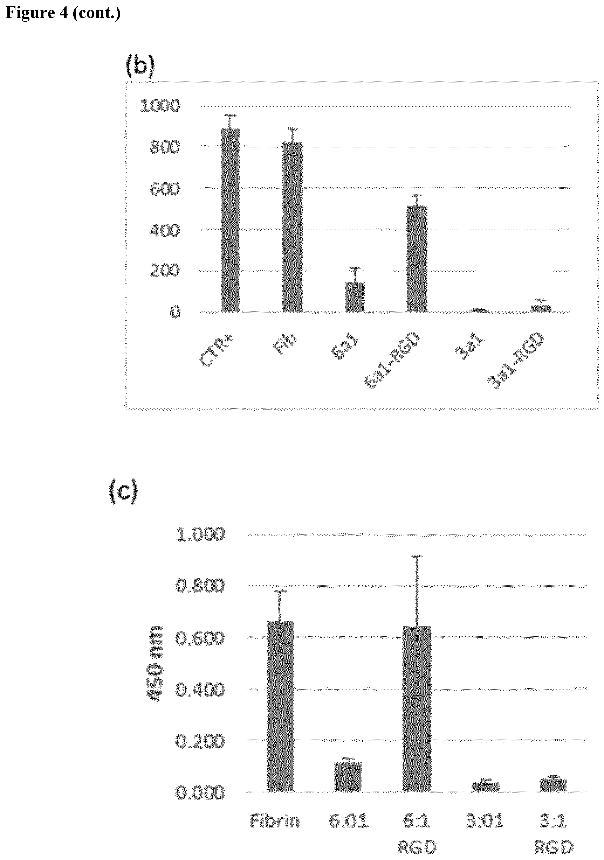

For the analysis of the ex vivo biocompatibility of the hydrogels, we carried out 2D cell cultures of fibroblasts and put them in contact with the different experimental groups during 48 h. From the L/D analysis of the samples, it was clear that the inclusion of Fmoc-RGD in the fibrin-peptide hydrogel formulation resulted in a large increase in the number of living cells **(****Fig. 4a-****b).** On the contrary, the addition of Fmoc-FF alone resulted in a reduction of the number of living cells with respect to the positive cultures and to those in contact with fibrin hydrogels -remark that no statistically significant differences exist between the fibrin hydrogels and the positive control. Thus, the presence of Fmoc-RGD in the formulation of the fibrin-peptide hydrogels resulted in the rise of the number of living cells with respect to samples not containing Fmoc-RGD. With respect to the role of peptides, the best results for are obtained for those hydrogels in which peptides are in a small proportion (6:1) with respect to fibrin.

To further analyze the biocompatibility of the hydrogels, we carried out WST-1 assays based on the colorimetric transformation oftetrazolium salt (WST-1) to formazan driven by the activity of the mitochondrial dehydrogenase of living cells, which is directly proportional to the number of viable (i.e., metabolically active) cells. Results demonstrate that no statistically differences exist between fibrin hydrogels and fibrin peptide (6:1) hydrogels containing Fmoc-RGD **(****Fig. 4c****).** On the contrary, fibrin-peptide (6:1) hydrogels without Fmoc-RGD and fibrin-peptide hydrogels at (3:1) proportion considerably decreased the cells viability. Nevertheless, the inclusion of Fmoc-RGD enhances the cell viability in both cases under study. Again, the best results for fibrin-peptide hydrogels viability were obtained in the cases in which peptides were present in a small proportion compared with fibrin. Thus, the inclusion of a small amount of peptides in the hydrogels formulation creates a good environment for cells viability and a great enhancement on their mechanical properties. Note that not only biocompatibility, but also adequate internal structure and mechanical properties are considered essential characteristics of biomaterials [Draghi: Characterization of Polymeric Biomaterials. http://dx.doi.org/10.1016/B978-0-08-100737-2.00008-X].

## Claims

1. Method for the preparation of a hydrogel suitable to produce artificial tissues, which comprises combining the following components:
a. Fibrin, fibrinogen or any source of fibrin or fibrinogen such as the human plasma;
b. An antifibrinolytic agent;
c. A fibrinogen polymerisation inducing agent;
d. A peptide with the ability to self-aggregate to form β-sheets;
e. A peptide biological ligand; and
f. Water.

2. Method, according to claim 1, wherein the ratio between the fibrin or fibrinogen and the peptide components is at least 3:1.

3. Method, according to any of the previous claims, wherein the ratio between the fibrin or fibrinogen and the peptide components is 6:1.

4. Method, according to any of the previous claims, wherein peptide with the ability to self-aggregate to form β-sheets is Fmoc-FF and wherein the peptide biological ligand is Fmoc-RGD.

5. Method, according to any of the previous claims, wherein the fibrinogen polymerisation inducing agent is a clotting factor, thrombin, a source of calcium preferably a calcium salt most preferably calcium chloride, or any combination thereof.

6. Method, according to any one of the preceding claims, wherein the antifibrinolytic agent is tranexamic acid.

7. Method, according to any of the previous claims, further comprising the addition of a protein, being a peptide with a chain of amino acids having a minimum molecular weight of 4 kDa, preferably fibronectin.

8. Method, according to any of the previous claims, further comprising adding a sample of biological material, preferably a sample comprising a plurality of isolated cells, or culturing the cells inside the resulting product.

9. Biomaterial or artificial tissue obtainable by the method according to any one of claims 1 to 8.

10. Biomaterial or artificial tissue, according to claim 9, comprising fibrin or fibrinogen, a peptide with the ability to self-aggregate to form β-sheets and a peptide biological ligand.

11. Biomaterial or artificial tissue, according to any of the claims 9 or 10, wherein the ratio between the fibrin or fibrinogen and the peptide components is up to 3:1.

12. Biomaterial or artificial tissue, according to any of the claims 9 to 11, wherein the ratio between the fibrin or fibrinogen and the peptide components is 6:1.

13. Biomaterial or artificial tissue, according to any of the claims 9 to 12, wherein peptide with the ability to self-aggregate to form β-sheets is Fmoc-FF and wherein the peptide biological ligand is Fmoc-RGD.

14. Composition comprising the biomaterial or artificial tissue according to any of the claims 9 to 13, or implant comprising the biomaterial or artificial tissue according to any of the claims 9 to 13.

15. Biomaterial or artificial tissue of claims 9 to 13, composition of claim 14, or implant of claim 14, for use as a medicament, preferably for use in tissue or organ regeneration, more preferably for use in restoring or partially or fully replacing the functional activity of a diseased or damaged tissue or organ.
